# EUROPÄISCHE PATENTSCHRIFT

(11) **EP 0 243 320 B1**
(45) Veröffentlichungstag und Bekanntmachung des Hinweises auf die Patenterteilung: **09.06.1993**
(21) Anmeldenummer: 87810250.8
(22) Anmeldetag: 21.04.1987
(51) Int. Cl.: C07D 213/16

(54) **Verfahren zur Entwässerung von Gemischen aus Wasser und N-heterocyclischen Verbindungen**
Process for the dehydratation of mixtures of water and N-heterocyclic compounds
Procédé pour la déshydratation de mélanges d'eau et de composés N-hétérocycliques

(30) Priorität: 25.04.1986 CH 1691/86
(43) Veröffentlichungstag der Anmeldung: 28.10.1987
(73) Patentinhaber: CIBA-GEIGY AG, 4002 Basel (CH)
(72) Erfinder: Blattner, Rudolf. Dr., D-7888 Rheinfelden 4 (DE); Hungerbühler, Konrad, Dr., CH-4451 Wintersingen (CH)

(56) Entgegenhaltungen:
- DD-A- 63 492

## Beschreibung

Gegenstand der Erfindung ist ein Verfahren zur Entwässerung von Gemischen aus Wasser und N-heterocyclischen Verbindungen durch Extraktion mittels wässriger Alkalihydroxid-Lösungen.

In der GB-A 580,048 ist ein Verfahren zur Trennung verschiedener Pyridinderivate aus ihren Gemischen durch Zugabe von Wasser und fraktionierte Destillation der einzelnen Wasserazeotrope beschrieben, wobei die einzelnen Wasserazeotrope durch Zugabe von festem Natriumhydroxid oder hochkonzentrierter Natronlauge (vorzugsweise 70%) und nachfolgender Trennung der entstehenden Phasen in Pyridinderivat und Wasser getrennt werden. Der Nachteil dieses Verfahrens besteht im relativ hohen Verbrauch an Natronlauge infolge der einstufigen Zugabe und in dem Umstand, dass die sich bildende verdünnte Lauge immer noch relativ hoch konzentriert sein muss, um den Wassergehalt in der Pyridinphase möglichst niedrig zu halten.

Die US-A 2,717,232 beschreibt ein kontinuierliches Verfahren zur Entwässerung von Pyridin-Wasser-Gemischen unter Zuhilfenahme von Natronlauge als Verdrängungs- und Benzol als Extraktionsmittel. Das erhaltene Pyridin-Benzolgemisch wird anschliessend durch fraktionierte Destillation getrennt.

Ein ähnliches Verfahren beschreibt die US-A 3,804,722, wobei die destillative Trennung von Pyridin und Wasser durch Zugabe von Natronlauge und Bisphenol erfolgt.

Beide Verfahren sind technisch aufwendig, energieintensiv und schaffen ökologische Probleme.

Aus der DD-A-63 492 ist ein Laborverfahren bekannt, das mit festem Ätznatron, bei geringerer Durchflussgeschwindigkeit und höherem Restgehalt an Basen in der Lauge, arbeitet.

Es bestand die Aufgabe, ein Verfahren zur Abtrennung von Wasser aus wasserhaltigen Gemischen von N-heterocyclischen Verbindungen, vorzugsweise solchen von Pyridin und Pyridinderivaten, zu finden, welches einfach ist und die Gemische weitgehend entwässert.

Diese Aufgabe löst das erfindungsgemässe Verfahren zur Entwässerung von Gemischen aus Wasser und N-heterocyclischen Verbindungen durch Extraktion mit wässrigen Alkalien im Gegenstrom, dadurch gekennzeichnet, dass die Gegenstromextraktion kontinuierlich durchgeführt wird, mittels 40 bis 60%iger wässriger Natriumhydroxid-Lösungen.

Vorzugsweise führt man das Verfahren mehrstufig nach dem Mixer-Settler-Prinzip durch.

Bei den N-heterocyclischen Verbindungen, deren Rückgewinnung durch das erfindungsgemässe Verfahren vorgesehen ist, handelt es sich um Verbindungen, die innerhalb des Temperaturbereichs von 20 bis 100°C in flüssiger Phase vorliegen. Dazu gehören vorzugsweise die nur ein Stickstoffatom im Ring aufweisenden Verbindungen wie Pyridin und dessen Derivate und Homologe, beispielsweise Picoline, Lutidine, Collidin, Piperidin, Morpholin und Chinolin. Insbesondere aber ist das erfindungsgemässe Verfahren zur Entwässerung von Gemischen aus Wasser und Pyridin geeignet, wobei das Mischungsverhältnis beliebig sein kann. Aus kostensparenden Gründen ist man aber bestrebt, von keinem zu hohen Wassergehalt auszugehen und entwässert daher zweckmässigerweise die nach einer Wasserdampf- oder Azeotropdestillation erhältlichen Gemische, die etwa 30 bis 70 % Wasser enthalten.

Die Natriumhydroxid-Lösung kann so lange im Gegenstrom verdünnt werden, bis aufgrund der zu niedrigen Alkalikonzentration eine Trennung zwischen dem N-Heterocyclus und der wässrigen alkalischen Phase nicht mehr möglich ist (Pyridin ∼ 8 %). Dann kann man die wässrige Natriumhydroxid-Lösung entweder aufkonzentrieren und erneut in den Kreislauf der Gegenstromextraktion bringen oder einer anderen Verwendung zuführen. Beispielsweise kann die aus der Gegenstromextraktion erhaltene verdünnte Natronlauge ohne weitere Reinigung von geringen Resten und N-heterocyclischen Verbindungen, wie insbesondere Pyridin, zur Aufarbeitung von Reaktionsmassen eingesetzt werden, wie sie zum Beispiel bei den Aluminiumchlorid/Pyridin-Schmelzen zur Herstellung von Küpenfarbstoffen anfallen.

Für die Gegenstromextraktion verwendet man die bekannten Geräte bzw. Anlagen, wie beispielsweise Rührkolonnen, Siebbodenkolonnen bzw. pulsierte Siebbodenkolonnen, Füllkörperkolonnen oder Mixer-Settler-Apparate. Während des Extrahierens steigt die Temperatur durch die freiwerdende Verdünnungswärme an. Die Menge der Verdünnungswärme ist abhängig von der Laugenkonzentration und dem Wassergehalt der zu entwässernden Gemische, wobei eine Kühlung normalerweise nicht erforderlich ist, wenn der Temperaturanstieg nicht über den Siedepunkt des Gemisches bzw. der leichter flüchtigen Komponente hinausgeht.

In den nachfolgenden Beispielen beziehen sich die Prozentangaben auf Gewichtsprozente.

Beispiel 1: In einer Extraktionskolonne (Rührkolonne mit 12 Böden) werden 2,5 kg/Stunde einer wässrigen Pyridinlösung (30 % Pyridin) mit 0,75 kg/Stunde einer 50%igen wässrigen Natronlauge im Gegenstrom extrahiert. Die Temperatur steigt dabei durch die Verdünnungswärme auf etwa 70°C an.

Erhalten werden 0,73 kg/Stunde Pyridin (99,2 % Pyridin und 0,8 % Wasser) und 2,52 kg/Stunde einer 17%igen wässrigen Natronlauge.

Die erhaltene Natronlauge enthält noch 0,8% Pyridin, das in einer Strippkolonne entfernt und dann erneut in die Extraktion eingespeist werden kann.

Beispiel 2: Entsprechend der Methode von Beispiel 1 werden in die Zuläufe der Gegenstromkolonne 2,5 kg/Stunde eines Gemisches aus Pyridin und Wasser (50 % Pyridin) und 0,83 kg/Stunde einer 50%igen wässrigen Natronlauge gegeben, wobei die Temperatur auf etwa 60°C ansteigt.

Erhalten werden 1,23 kg/Stunde Pyridin (99,2 % Pyridin und 0,8 % Wasser) und 2,1 kg/Stunde einer 20%igen wässrigen Natronlauge, die 0,9 % Pyridin enthält.

Die erhaltene Natronlauge kann ohne weitere Reinigung von Pyridin zur Aufarbeitung von Reaktionsmassen verwendet werden, wie sie z.B. bei den AlCl₃/Pyridin-Schmelzen zur Herstellung von Küpenfarbstoffen anfallen.

## Patentansprüche

1. Verfahren zur Entwässerung von Gemischen aus Wasser und N-heterocyclischen Verbindungen durch Extraktion mit wässrigen Alkalien im Gegenstrom, gekennzeichnet dadurch, dass die Gegenstromextraktion kontinuierlich durchgeführt wird. mittels 40 bis 60%iger wässriger Natriumhydroxid-Lösungen.

2. Verfahren gemäss Anspruch 1, gekennzeichnet durch mehrstufige Gegenstromextraktion nach dem Mixer-Settler-Prinzip.

3. Verfahren gemäss einem der vorangehenden Ansprüche 1 oder 2, gekennzeichnet durch kontinuierliche Gegenstromextraktion von Gemischen aus Wasser und Pyridin oder Pyridinderivaten.

4. Verfahren gemäss Anspruch 1, dadurch gekennzeichnet, dass die wässrige Alkalihydroxid-Lösung nach erfolgter Gegenstromextraktion aufkonzentriert und im Kreislauf geführt wird.

5. Verfahren gemäss Anspruch 1, gekennzeichnet durch kontinuierliche Gegenstromextraktion bei Temperaturen zwischen 20 und 100°C.

6. Verwendung der nach dem Verfahren gemäss Anspruch 1 erhältlichen verdünnten alkalischen Lösungen zur Aufarbeitung von Reaktionsmassen, die bei den Aluminiumchlorid/Pyridin-Schmelzen zur Herstellung von Küpenfarbstoffen anfallen.

## Claims

1. Process for dehydrating mixtures of water and N-heterocyclic compounds by extraction with aqueous alkalis in counter-current, characterised in that the counter-current extraction is carried out continuously using 40 to 60% aqueous sodium hydroxide solutions.

2. Process according to claim 1, characterised by multiple stage counter-current extraction by the mixer-settler principle.

3. Process according to either of the preceding claims 1 or 2, characterised by continuous counter-current extraction of mixtures of water and pyridine or pyridine derivatives.

4. Process according to claim 1, characterised in that the aqueous alkali metal hydroxide solution, after counter-current extraction has been completed, is concentrated and circulated.

5. Process according to claim 1, characterised by continuous counter-current extraction at temperatures between 20 and 100°C.

6. Use of the dilute alkaline solutions, obtainable by the process according to claim 1, for the work-up of reaction mixtures, which are produced in the aluminium chloride/pyridine melts for the preparation of vat dyes.

## Revendications

1. Procédé de déshydratation de mélanges d'eau et de composés N-hétérocycliques par extraction à contre-courant avec des solutions aqueuses d'alcalis, **caractérisé** en ce que l'on réalise l'extraction à contre-courant en continu, au moyen de solutions aqueuses d'hydroxyde de sodium, à 40 à 60 %.

2. Procédé selon la revendication 1, **caractérisé** par une extraction à contre-courant à plusieurs étages, selon le principe mélangeur-décanteur.

3. Procédé selon la revendication 1 ou 2, **caractérisé** par une extraction à contre-courant en continu de mélanges d'eau et de pyridine ou de dérivés de la pyridine.

4. Procédé selon la revendication 1, **caractérisé** en ce que la solution aqueuse d'hydroxyde alcalin, obtenue une fois effectuée l'extraction à contre-courant, est concentrée et introduite dans le circuit.

5. Procédé selon la revendication 1, **caractérisé** par une extraction à contre-courant en continu, à une température comprise entre 20 et 100°C.

6. Utilisation des solutions alcalines diluées, que l'on peut obtenir par le procédé selon la revendication 1, pour le traitement de masses réactionnelles qui se présentent dans le cas des masses fondues chlorure d'aluminium/pyridine pour la préparation de colorants de cuve.
